# EUROPEAN PATENT APPLICATION

(11) **EP 4 219 732 A2**
(43) Date of publication of application: **02.08.2023**
(21) Application number: 22214693.8
(22) Date of filing: 26.04.2019
(51) Int. Cl.: C12P 7/6472, C12N 1/12, C11B 1/10

(54) **MICROBIAL OIL AND METHOD FOR PRODUCING MICROBIAL OIL**

(30) Priority: 26.04.2018 JP 2018085751
(62) Divisional of application: 19792171.1
(71) Applicant: Nissui Corporation, Tokyo 105-8676 (JP)
(72) Inventor: SEKIGUCHI, Takayoshi, Tokyo, 1920991 (JP); SATO, Seizo, Tokyo, 1920991 (JP); ISHIWATA, Yuko, Tokyo, 1920991 (JP)
(74) Representative: Mewburn Ellis LLP

(57) **Abstract**

To provide a microbial oil that contains eicosapentaenoic acid (EPA) at a high concentration and can effectively exert the function of EPA. Microbial oils are disclosed containing EPA and docosahexaenoic acid (DHA), in which the content of EPA is 38% by area or greater relative to the total fatty acids, and which have at least one fatty acid ratio selected from:
(a) a ratio of the content of EPA to the content of DHA of 1.7 or more;
(b) a ratio of the content of fatty acid(s) having 18 carbon atoms to the content of fatty acid(s) having 20 carbon atoms of 0.3 or less;
(c) a ratio of the content of fatty acid(s) having 18 carbon atoms to the content of fatty acid(s) having 22 carbon atoms of 1.5 or less.

## Description

### TECHNICAL FIELD

The present disclosure relates to a microbial oil and a method for producing a microbial oil.

### BACKGROUND ART

Eicosapentaenoic acid (which hereinafter may be abbreviated as EPA), docosahexaenoic acid (which hereinafter may be abbreviated as DHA), and others, which are n-3 polyunsaturated fatty acids, have various physiological actions and are used as, for example, medicines, health foods, and food materials. Beverages including EPA-containing oil are also approved as foods for specified health uses. Demand for n-3 polyunsaturated fatty acids as supplements is very high both in Japan and overseas. Accordingly, there has been a demand for the production of polyunsaturated fatty acids with high purity in large quantities.

In order to industrially efficiently produce EPA, which is one of such n-3 polyunsaturated fatty acids, a method of concentrating EPA in an EPA-containing oil to a high concentration using a distillation technique or the like is used. It has also been proposed to obtain an EPA-rich oil as a raw material oil before application of such a concentration technique.

For example, Patent Document 1 discloses a method for transforming Stramenopiles capable of producing a microbial oil having unsaturated fatty acids content increased by a gene recombination technique.

Patent Document 2 discloses a method for producing oil concentrated in unsaturated fatty acids, which includes subjecting an oil or fat containing unsaturated fatty acids to a hydrolysis reaction with lipase, and separating a glyceride fraction containing concentrated unsaturated fatty acids, in which calcium hydroxide, magnesium chloride, ammonium sulfate, calcium carbonate, potassium dihydrogen phosphate, or ammonium chloride is added as a reaction additive for the hydrolysis reaction with lipase.

### CITATION LIST

### Patent Literature

Patent Document 1: JP 2016-189787 A
Patent Document 2: JP 2013-55893 A

### SUMMARY OF THE INVENTION

### Technical Problem

Even if the microbial oil contains EPA at a high concentration, if saturated or unsaturated fatty acids other than EPA are present in the microorganism, a large amount of microbial oil is required to obtain EPA with high purity, and productivity tends to be inferior. For example, microorganisms that produce both EPA and DHA include microorganisms that tend to produce more DHA than EPA. It is industrially disadvantageous to utilize such microorganisms for obtaining microbial oils containing EPA. In addition, although fatty acids often have specific functionalities, plural fatty acids do not always act synergistically with each other.

An object of the present disclosure is to provide a microbial oil that contains EPA at a high concentration and can effectively exert the function of EPA, and a method for producing the same.

Another object of the present disclosure is to expand the selection range of microbial species that can be used to efficiently obtain EPA-containing compositions such as EPA-containing microbial oils.

### Solution to the Problem

The present disclosure includes the following.
[1] A microbial oil comprising eicosapentaenoic acid (EPA) and docosahexaenoic acid (DHA), the content of eicosapentaenoic acid being 38 area% or greater based on the total fatty acids, and the microbial oil having at least one fatty acid ratio selected from the group consisting of the following (a) to (c):
   (a) a ratio of the content of EPA to a content of DHA of 1.7 or higher;
   (b) a ratio of a content of the fatty acids having 18 carbon atoms to a content of the fatty acids having 20 carbon atoms of 0.3 or less; and
   (c) a ratio of the content of the fatty acids having 18 carbon atoms to a content of the fatty acids having 22 carbon atoms of 1.5 or less.
[2] The microbial oil according to [1], in which a content of triglyceride is 70% by weight or more based on a total weight of the microbial oil.
[3] The microbial oil according to [1] or [2], in which a content of saturated fatty acids is 40 area% or less based on the total fatty acids.
[4] The microbial oil according to any one of [1] to [3], in which a content of arachidonic acid (ARA) is 10.0 area% or less based on the total fatty acids.
[5] The microbial oil according to any one of [1] to [4], in which a content of arachidonic acid (ARA) is 5.0 or less based on the content of EPA.
[6] The microbial oil according to any one of [1] to [5], in which a content of docosahexaenoic acid is lower than the content of eicosapentaenoic acid.
[7] The microbial oil according to any one of [1] to [6], in which a content of docosahexaenoic acid is 0.5 area% or more and 25 area% or less based on the total fatty acids.
[8] The microbial oil according to any one of [1] to [7], in which a content of n-6 docosapentaenoic acid (n-6DPA) is 2.0 or less based on a content of n-3 docosapentaenoic acid (n-3DPA).
[9] The microbial oil according to any one of [1] to [8], in which a content of n-3 docosapentaenoic acid (n-3DPA) is 0.3 area% or more based on the total fatty acids.
[10] The microbial oil according to any one of [1] to [9], in which a content of eicosatetraenoic acid (ETA) is 0.1 area% or greater based on the total fatty acids.
[11] The microbial oil according to any one of [1] to [10], in which a total content of unsaturated fatty acids having 18 carbon atoms is 2.0 area% or less based on the total fatty acids.
[12] The microbial oil according to any one of [1] to [11], which is a crude oil.
[13] A method for producing a microbial oil including preparing a microorganism having an ability to produce both eicosapentaenoic acid and docosahexaenoic acid, culturing the prepared microorganism in a medium containing glycerol under an environment aerated at 30 mL/min or more and 1500 mL/min or less, and obtaining the microbial oil described in any one of claims 1 to 12 from biomass obtained after culturing.
[14] The method for producing a microbial oil according to [13], in which the microorganism is at least one microorganism selected from the group consisting of microorganisms belonging to Stramenopiles.
[15] The method for producing a microbial oil according to any one of [13] or [14], in which the microorganism does not grow, or can produce more docosahexaenoic acid than eicosapentaenoic acid, in a medium containing glucose.
[16] The method for producing a microbial oil according to any one of [13] to [15], in which the culture environment is an environment equivalent to a water evaporation amount of 0.5 mL when the culture is left at 30°C for 10 days.
[17] A microbial oil obtained by the production method described in any one of [13] to [16].
[18] A method for controlling a fatty acid content ratio of a microbial oil, including preparing a microorganism that does not grow, or can produce a microbial oil containing more docosahexaenoic acid than eicosapentaenoic acid, in a medium containing glucose, culturing the prepared microorganism in a culture medium containing glycerol under an environment aerated at 30 mL/min or more and 1500 mL/min or less, and obtaining a microbial oil containing more eicosapentaenoic acid than docosahexaenoic acid from biomass obtained after culturing.

### ADVANTAGEOUS EFFECTS OF THE INVENTION

According to the present disclosure, it is possible to provide a microbial oil containing EPA at a high concentration and capable of effectively exerting the function of EPA, and a method for producing the same. According to the present disclosure, the selection range of microbial species that can be used to efficiently produce an EPA-containing composition such as an EPA-containing microbial oil can be expanded.

### DESCRIPTION OF EMBODIMENTS

A microbial oil according to the present disclosure includes eicosapentaenoic acid (EPA) and docosahexaenoic acid (DHA), the content of eicosapentaenoic acid being 38 area% or more based on the total fatty acids, and the microbial oil having at least one fatty acid ratio selected from the group consisting of the following (a) to (c):
(a) the ratio of EPA content to the content of DHA of 1.7 or higher;
(b) a ratio of the content of fatty acid having 18 carbon atoms to the content of fatty acid having 20 carbon atoms of 0.3 or less; and
(c) a ratio of the content of fatty acid having 18 carbon atoms to the content of fatty acid having 22 carbon atoms of 1.5 or less.

Since the microbial oil according to the present disclosure contains EPA in an amount of 38 area% or greater based on the total fatty acids and has at least one of the fatty acid ratios (a) to (c) described above, the desired function of EPA can be effectively exerted.

Specifically, when the microbial oil of the present disclosure has a ratio of the content of EPA to the content of DHA of a certain level or more, the desired function of EPA can be effectively exerted. Further, since the microbial oil contains DHA together with EPA, a synergistic effect of DHA and EPA can be expected.

A saturated or unsaturated fatty acid having 18 carbon atoms is known as a precursor of a polyunsaturated fatty acid having 20 or more carbon atoms. Thus, in the microbial oil of the present disclosure, when the content of saturated or unsaturated fatty acid having 18 carbon atoms is in a certain ratio or less relative to the content of fatty acid having 20 carbon atoms or fatty acid having 22 carbon atoms, it is efficiently used to obtain EPA and DHA, and the microbial oil can contain EPA and DHA with high purity. In the microbial oil of the present disclosure, the content of fatty acid having 18 carbon atoms is suppressed within a predetermined range, or the content of DHA that can be produced together is within a predetermined range, so that more efficient functioning of EPA can be expected.

In the present specification, the microbial oil according to the present disclosure that contains EPA and DHA, has an EPA content of 38 area% or more based on the total fatty acids, and has predetermined requirements, may be referred to as "EPA-rich microbial oil".

The method for producing a microbial oil according to the present disclosure includes preparing a microorganism having an ability to produce both eicosapentaenoic acid and docosahexaenoic acid, culturing the prepared microorganism in a medium containing glycerol under an environment aerated at 30 mL/min or more and 1500 mL/min or less, and obtaining the EPA-rich microbial oil of the present disclosure from biomass obtained after culturing.

The method for producing a microbial oil according to the present disclosure includes culturing a microorganism capable of producing EPA and DHA in a medium containing glycerol under a predetermined aerated environment. As a result, the EPA-rich microbial oil according to the present disclosure can be efficiently obtained.

The glycerol-containing culture medium used in the method for producing a microbial oil according to the present disclosure may contain glucose at a glucose concentration at which the microorganism grows and the content of eicosapentaenoic acid in the microbial oil is higher than the content of docosahexaenoic acid.

In the present specification, the term "step" includes not only an independent step but also a step that cannot be clearly distinguished from other steps provided that an intended object of the step is achieved. In the present specification, numerical ranges indicated by "to" are ranges including the numerical values described before and after the "to" as the minimum and maximum values, respectively. In the present specification, when a mixture contains a plurality of substances that correspond to each component, unless otherwise specified, the content of each component in the mixture means the total content of the plurality of substances present in the mixture. In the present specification, the term "not more than" or "less than" with regard to a percentage includes 0%, that is, "not containing", or means a range including a value undetectable by existing means, unless a lower limit value is specifically stated.

The term "biomass" means an aggregation or lump of microorganisms or cells at a certain point of time during growth in a certain region or in an ecosystem. The region or ecosystem may be a naturally occurring environment (for example, aquatic) or a synthetic environment (for example, open or closed) fermentor or bioreactor).

In the present disclosure, a"microbial oil" is a mixture of organic substances that is obtained from microbial biomass and is insoluble in water at room temperature and atmospheric pressure, that is, at 25°C and 1 atmospheric pressure. The microbial oil contains oily components, such as a saturated or unsaturated fatty acid, phospholipid, sterol, glycerol, ceramide, sphingolipid, terpenoid, flavonoid, or tocopherol, and the saturated or unsaturated fatty acid may also be present as a constituent fatty acid in another oily component. In the present specification, the term "oil" means a composition containing these lipids and oily components.

The term "fatty acid" indicates not only a free saturated or unsaturated fatty acid itself, but also includes fatty acids contained as constituent units in free saturated or unsaturated fatty acids, saturated or unsaturated fatty acid alkyl esters, triglycerides, diglycerides, monoglycerides, phospholipids, steryl esters, and the like, which can also be called constituent fatty acids. In the present specification, unless otherwise noted, the forms of compounds containing fatty acids may be omitted. Examples of forms of compounds containing fatty acids may include a free fatty acid form, a fatty acid alkyl ester form, a glyceryl ester form, a phospholipid form, and a steryl ester form. Compounds containing the same fatty acids may be contained in a single form or may be contained as a mixture of two or more forms in the oil.

It has been empirically determined that the reaction efficiency of hydrolysis or alcoholysis of fatty acids is high, and after hydrolysis or alcoholysis, a composition is obtained containing mainly free fatty acids or lower alcohol esters thereof as the fatty acid forms. For this reason, unless otherwise specified, fatty acids after the processing step are denoted without mentioning that they are a composition or that the fatty acid is in a free fatty acid form or a lower alcohol ester form. However, this does not exclude the possibility that fatty acids in a form other than free fatty acid form or lower alcohol ester form are contained.

For denoting fatty acids, a numerical expression may be used in which the number of carbons, the number of double bonds, and the locations of double bonds are each expressed in a simplified manner using numbers and alphabets, respectively. For example, a saturated fatty acid having 20 carbon atoms is expressed as "C20:0", and a tri-unsaturated unsaturated fatty acid having 20 carbon atoms and three double bonds in the carbon chain is expressed as "C20:3". For example, eicosapentaenoic acid can be expressed as "C20:5,n-3", and docosahexaenoic acid as "C22:6,n-3". The "n-" indicates the position of the first double bond counted from the methyl end of the fatty acid. For example, "n-6" indicates that the position of the double bond is the sixth position counted from the methyl end of the fatty acid, and "n-3" indicates that the position of the double bond is the third position from the methyl end of the fatty acid. This method is known to those of ordinary skill in the art, and those of ordinary skill in the art can easily specify fatty acids expressed in accordance with this method.

In this context, the expression of a combination of carbon number and the term "fatty acid" such as "fatty acid having 20 carbon atoms" may be used asa generic term to refer to saturated fatty acids having the above carbon number and mono- or poly-saturated fatty acids having the above carbon number. For example, the term "fatty acid having 20 carbon atoms" includes saturated fatty acids having 20 carbon atoms, mono-unsaturated fatty acids having 20 carbon atoms, and di- or poly-unsaturated fatty acids having 20 carbon atoms.

In the present specification, polyunsaturated fatty acid having 20 or more carbon atoms may be referred to as "PUFA" unless otherwise specified. The polyunsaturated fatty acids having at least 20 carbon atoms in PUFA include di- or polyunsaturated fatty acids, preferably tri- or poly-unsaturated fatty acids. The number of carbon atoms of the polyunsaturated fatty acid refers to the number of carbon atoms of the constituent fatty acids. Examples of polyunsaturated fatty acids having at least 20 carbon atoms may include polyunsaturated fatty acids having from 20 to 22 carbons, specifically, eicosadienoic acid (C20:2, n-6, EDA), dihomo-γ-linolenic acid (C20:3, n-6, DGLA), mead acid (C20:3, n-9, MA), eicosatetraenoic acid (C20:4, n-3, ETA), arachidonic acid (C20:4, n-6, ARA), eicosapentaenoic acid (C20:5, n-3, EPA), docosatetraenoic acid (C22:4, n-6, DTA), docosapentaenoic acid (C22:5, n-3, DPA), docosapentaenoic acid (C22:5, n-6, DPA), and docosahexaenoic acid (C22:6, n-3, DHA). In the present specification, among PUFAs, an n-3 PUFA may be particularly referred to as "n-3PUFA", and an n-6 PUFA may be referred to as "n-6PUFA".

In the present specification, the content of fatty acids with respect to the total weight of fatty acids in the oil in the present specification is determined based on the fatty acid composition. The fatty acid composition may be determined by the usual method. Specifically, the oil to be analyzed is esterified with a lower alcohol and a catalyst to obtain fatty acid lower alcohol esters. Thereafter, the obtained fatty acid lower alcohol esters are analyzed using gas chromatography. Peaks corresponding to each of the fatty acids are identified in the chromatogram obtained by gas chromatography, and the peak area of each of the fatty acids is determined using the Agilent ChemStation integration algorithm (revision C.01.03[37], Agilent Technologies). The "peak area" indicates a ratio (area percent) of the peak area for respective components to the area of all peaks as determined in the chromatogram of the oil having various fatty acids as constituent components as obtained by analysis of gas chromatography, thin-layer chromatography/flame ionization detector (TLC/FID), or the like, and indicates the content ratio of the component of the peak. The value of the area percent obtained by the measurement method described above is the same as the value of the weight percent of each fatty acid in a sample, and may be used interchangeably. Refer to "Standard Methods for the Analysis of Fats, Oils, and Related Materials", 2013 Edition, 2.4.2.1-2013 Fatty Acid Composition (FID constant temperature gas chromatography) and 2.4.2.2-2013 Fatty Acid Composition (FID elevated temperature gas chromatography) established by the Japan Oil Chemists' Society (JOCS).

Hereinafter, the present disclosure will be described.

### Microbial oil

The EPA-rich microbial oil according to one embodiment of the present disclosure contains eicosapentaenoic acid (EPA) and docosahexaenoic acid (DHA), the content of eicosapentaenoic acid being 38 area% or greater based on all fatty acids, and the microbial oil having at least one fatty acid ratio selected from the group consisting of the following (a) to (c):
(a) the ratio of the content of EPA to the content of DHA of 1.7 or higher;
(b) the ratio of the content of the fatty acids having 18 carbon atoms to the content of the fatty acids having 20 carbon atoms of 0.3 or less; and
(c) the ratio of the content of the fatty acids having 18 carbon atoms to the content of the fatty acids having 22 carbon atoms of 1.5 or less.

The content of EPA in the EPA-rich microbial oil is 38 area% or more, from the viewpoints of exerting the function of EPA, purification efficiency, and others, and may be 38.5 area% or greater, 39 area% or greater, 39.5 area% or greater, 40 area% or greater, 40.5 area% or greater, 41 area%, 41.5 area% or greater, 42 area% or greater, 42.5 area% or greater, 43 area% or greater, 43.5 area% or greater, 44 area% or greater, 44.5 area% or greater, 45 area% or greater, 45.5 area% or greater, 46 area%, 46.5 area% or greater, 47 area% or greater, 47.5 area% or greater, 48 area% or greater, 48.5 area% or greater, 49 area%, 49.5 area% or greater, or 50 area% or greater. The upper limit value of EPA in the EPA-rich microbial oil is not particularly limited, and may be, for example, 99.5 area% or less, 99.3 area% or less, 99 area% or less, 98.5 area% or less, 98 area% or less, or 97 area% or less. The range of the content of EPA in the EPA-rich microorganism may be any combination of these upper and lower limits.

The EPA-rich microbial oil contains EPA with DHA, and the content of DHA is preferably lower than the content of EPA from the viewpoints of exerting the function of EPA in the EPA-rich microbial oil and the purification efficiency of EPA.

The content of DHA in the EPA-rich microbial oil may be 0.5 area% or greater, 0.7 area% or greater, 1 area% or greater, 1.5 area% or greater, 2 area% or greater, or 3 area% or greater, from the viewpoint of exerting the function of EPA, the function of DHA, and the synergistic effect of EPA and DHA. The upper limit of DHA in EPA-rich microbial oil may be 25 area% or less, 22 area% or less, 20 area% or less, 18 area% or less, 15 area% or less, 13 area% or less, 12 area% or less, 11.5 area% or less, or 11 area% or less, from the viewpoint of the purification efficiency of EPA. The range of the content of DHA in the EPA-rich microorganism may be any combination of these upper and lower limits, and may be, for example, 0.5 area% or more and 25 area% or less, or 1.5 area% or more and 15 area% or less.

The EPA-rich microbial oil may or may not contain saturated or unsaturated fatty acids other than EPA and DHA. The content of each of the saturated or unsaturated fatty acids other than EPA and DHA in the EPA-rich microbial oil may be 0 area% or greater, from the viewpoint of the functions and characteristics of each fatty acid, or from the viewpoint of exerting the function of the EPA-rich microbial oil. Examples of the saturated or unsaturated fatty acids other than EPA and DHA may include saturated fatty acids with any carbon number, mono-unsaturated fatty acids having 18 carbon atoms, di-unsaturated fatty acids having 18 carbon atoms, unsaturated fatty acids having 20 carbon atoms other than EPA, unsaturated fatty acids having 21 carbon atoms, unsaturated fatty acids having 22 carbon atoms other than DHA, and other unsaturated fatty acids.

The EPA-rich microbial oil according to an embodiment of the present disclosure may have at least one selected from the group consisting of the following fatty acid contents and ratios, from the viewpoints of effectively exerting the function of EPA, purification efficiency of a high-purity EPA-containing composition, and obtaining at least one of the advantages described below.
(a) In the EPA-rich microbial oil, the ratio of the content of EPA to the content of DHA (EPA/DHA) may be 1.7 or higher, 1.8 or higher, 1.9 or higher, 2.0 or higher, 2.2 or higher, 2.5 or higher, 2.7 or higher, 3.0 or higher, 3.2 or higher, 3.5 or higher, 3.7 or higher, 4.0 or higher, 4.2 or higher, 4.4 or higher, 4.5 or higher, 4.7 or higher, or 5.0 or higher, from the viewpoint of exerting the function of EPA, purification efficiency, and others. The upper limit of the EPA/DHA ratio is not particularly limited, and may be, for example, 10.0 or less, or 8.0 or less. In the EPA-rich microorganism, the range of the EPA/DHA ratio may be any combination of these upper and lower limits, and may be, for example, 1.7 or higher and 10.0 or less, or 3.0 or more and 8.0 or less.
(b) In the EPA-rich microbial oil, the content of fatty acids having 18 carbon atoms may be 0.3 or less, 0.1 or less, 0.05 or less, 0.03 or less, or 0.02 or less relative to the content of fatty acids having 20 carbon atoms, from the viewpoints of the purity of EPA, exerting the function of EPA, and others.
(c) In the EPA-rich microbial oil, the content of fatty acids having 18 carbon atoms may be 1.5 or less, 1.0 or less, 0.5 or less, 0.1 or less, 0.08 or less, or 0.05 or less relative to the content of fatty acid having 22 carbon atoms, from the viewpoint of effectively exerting the synergistic effect of DHA with EPA.

The EPA-rich microorganism may contain no fatty acid having 18 carbon atoms, and thus the lower limit of the content of the fatty acid having 18 carbon atoms may be 0 area%.

The EPA-rich microorganism has at least one fatty acid ratio selected from the group consisting of (a) to (c) above, and thus has advantages such as purity of EPA, exerting of the function of EPA, purification efficiency, and others.

The EPA-rich microorganism may have at least one fatty acid content or ratio selected from the group consisting of (d) to (n) below, in addition to (a) to (c).

(d) The content of saturated fatty acids having any carbon number (hereinafter simply referred to as saturated fatty acids) may be 40 area% or less, 30 area% or less, 25 area% or less, 20 area% or less, 19% or less, 18 area% or less, 17 area% or less, or 15 area% or less based on the total fatty acids. An EPA-rich microbial oil having saturated fatty acids content in this range can have advantages such as better stability at low temperatures and superior handleability.

(d') In the EPA-rich microbial oil, the ratio of the content of saturated fatty acids to EPA may be 1.5 or less and 1.1 or less, 0.8 or less, 0.5 or less, or 0.4 or less, from the viewpoints of achieving good stability of PUFA and excellent handleability at low temperatures.

Examples of the saturated fatty acid that can be contained in the EPA-rich microbial oil may include saturated fatty acids having 12 or more carbon atoms. Examples of saturated fatty acids having 12 or more carbon atoms may include lauric acid (C12:0), myristic acid (C14:0), palmitic acid (C16:0), stearic acid (C18:0), arachidic acid (C20:0), behenic acid (C22:0), and lignoceric acid (C24:0).

(e) Among the polyunsaturated fatty acids having 20 carbon atoms, the content of arachidonic acid (C20:4, n-6, ARA) may be 10.0 area% or less, 5.0 area% or less, 3.0 area% or less, 2.5 area% or less, or 2.0 area% or less based on the total fatty acids. When the content of arachidonic acid in the EPA-rich microbial oil is within this range, an EPA-rich microbial oil can obtain a superior effect considering the antagonistic action of n-6 PUFA.

(e') In the EPA-rich microbial oil, the ratio of the content of ARA to that of EPA may be 5.0 or less, 1.0 or less, 0.5 or less, 0.1 or less, 0.08 or less, 0.05 or less, or 0.04 or less, considering the antagonistic action of n-6 PUFA.

(f) Among the polyunsaturated fatty acids having 20 carbon atoms, the content of eicosatetraenoic acid (C20:4, n-3, ETA) may be 0.1 area% or more, 0.5 area% or more, or 1.0 area% or more based on the total fatty acids. When eicosatetraenoic acid is contained in the EPA-rich microbial oil at this content, the function of EPA, the function of DHA, the function of ETA, and the synergistic effect of EPA, DHA and ETA can be exerted. The upper limit of the content of ETA may be 10.0 area% or less, 5.0 area% or less, 3.0 area% or less, 2.0 area% or less, or 1.8 area relative to the content of EPA.

In the EPA-rich microorganism, the ETA content range may have any combination of these upper and lower limits, and may be, for example, 0.1 area% or more and 10.0 area% or less, or 0.5 area% or more and 5.0 area% or less.

(f) In the EPA-rich microbial oil, the ratio of the content of ETA to that of EPA may be 1.6 or less, 1.0 or less, or 0.05 or less, from the viewpoints of exerting the function of EPA, purification efficiency, and others. The lower limit of the ETA/EPA ratio is not particularly limited and may be, for example, 0.001 or higher or 0.005 or higher.

In the EPA-rich microorganism, the ETA/EPA ratio range may have any combination of these upper and lower limits, and may be, for example, 0.001 or higher and 1.6 or less, or 0.001 or higher and 1.0 or less.

(g) The EPA-rich microbial oil may contain, in addition to DHA, n-3 unsaturated fatty acids having 22 or more carbon atoms and a valence of 2 or more, preferably 3 or more. In the present specification, an n-3 unsaturated fatty acid having 22 or more carbon atoms and a valence of 2 or more may be referred to as n-3C22PUFA. Examples of n-3C22PUFA include docosapentaenoic acid (C22:5, n-3, n-3DPA) and DHA. The total content of n-3C22PUFAs in the EPA-rich microbial oil may be 0.3 area% or more, 0.5 area% or more, 1.0 area% or more, 5.0 area% or more, 8.0 area% or more, 10 area% or more, 15 area% or more, or 19% or more, and may be 35 area% or less, 30 area% or less, 25 area% or less, 23 area% or less, or 20 area% or less based on the total fatty acids, from the viewpoints of exerting the function of EPA, the function of DHA, the function of n-3C22PUFA, and the synergistic effect of EPA, DHA and n-3PUFA. In the EPA-rich microorganism, the range of the total content of PUFAs may havee any combination of these upper and lower limits, and may be, for example, 0.5 area% or more and 35 area% or less, or 10 area% or more and 25 area% or less.

(g') In the EPA-rich microbial oil, the ratio of the content of n-3C22PUFAs to that of EPA (n-3C22PUFAs/EPA) may be 3.0 or less, 2.0 or less, 1.0 or less, or 0.6 or less, from the viewpoints of exerting the function of EPA, the function of DHA, the function of n-3C22PUFA, and the synergistic effect of EPA, DHA, and n-3PUFA. In the EPA-rich microbial oil, the lower limit of the n-3C22PUFA/EPA ratio may be, for example, 0.01 or higher.

(h) The content of n-3DPA in the EPA-rich microbial oil may be 0.3 area% or greater, 1.0 area% or greater, 3.0 area% or greater, 5.0 area% or greater, or 8.0 area% or greater based on the total fatty acids. When n-3DPA is contained in the EPA-rich microbial oil at this content, the function of EPA, the function of DHA, the function of n-3DPA, and the synergistic effect of EPA, DHA and n-3DPA can be exerted. The upper limit of the content of n-3DPA may be 20.0 area% or less, 15.0 area%, or 12.0 area% or less relative to the content of EPA. In the EPA-rich microorganism, the content range of n-3DPA may have any combination of these upper and lower limits, and may be, for example, 0.3 area% or greater and 20.0 area% or less, or 8.0 area% or greater and 15.0 area% or less.

(h') In the EPA-rich microbial oil, the ratio of the content of n-3DPA to that of EPA should be 8.0 or less, 6.0 or less, or 5.0 or less, from the viewpoints of exerting the function of EPA and purification efficiency.

(i) The content of n-6 DPA in the EPA-rich microbial oil may be 2.0 area% or less, 1.0 area% or less, 0.5 area% or less, or 0.1 area% or less based on the total fatty acids. When the content of n-6DPA in the EPA-rich microbial oil is within this range, the EPA-rich microbial oil can obtain an effect equal to or higher than the antagonistic action of n-6PUFA.

(i') In the EPA-rich microbial oil, the ratio of the content of n-6PDA to that of EPA may be 1.0 or less, 0.1 or less, or 0.01 or less, considering the antagonistic action of n-6PUFA.

(j) In the EPA-rich microbial oil, the content of n-6DPA may be 2.0 or less, 1.0 or less, 0.1 or less, or 0.01 or less based on the content of n-3DPA, considering the antagonistic action of n-6PUFA.

(k) The EPA-rich microbial oil may contain mono- or poly-unsaturated fatty acids having 18 carbon atoms. The total content of unsaturated fatty acids having 18 carbon atoms in the EPA-rich microbial oil may be 2.0 area% or less, 1.0 area% or less, 0.5 area% or less, or 0.2 area% or less based on the total fatty acids, from the viewpoints of exerting EPA function and improving purification efficiency.

(k') In the EPA-rich microbial oil, the ratio of the total content of unsaturated fatty acids having 18 carbon atoms to the content of EPA may be 2.0 or less, 1.0 or less, 0.1 or less, 0.05 or less, 0.02 or less, or 0.01 or less, from the viewpoints of exerting EPA function and improving purification efficiency.

(l) In the EPA-rich microbial oil, the content of fatty acids having 21 carbon atoms may be 2.0 area% or less, 1.5 area% or less, or 1.0 area% or less based on the total fatty acids. An EPA-rich microbial oil having a content of fatty acids having 21 carbon atoms within the above range can have advantages such as better stability at low temperatures and more excellent handleability.

(l') In the EPA-rich microbial oil, the ratio of the content of fatty acid having 21 carbon atoms to that of EPA may be 1.0 or less, 0.1 or less, or 0.02 or less, from the viewpoints of stability at low temperatures and handleability.

(m) Among the polyunsaturated fatty acids having 20 carbon atoms, the content of dihomo-γ-linolenic acid (C20:3, n-6, DGLA) may be 5.0 area% or less, 1.0 area% or less, 0.5 area% or less, or 0.1 area% or less based on the total fatty acids. When the content of DGLA in an EPA-rich microbial oil is within this range, the EPA-rich microbial oil can achieve the same or superior effect considering the antagonistic action of n-6PUFA.

(m') In the EPA-rich microbial oil, the ratio of the content of the fatty acid DGLA to that of EPA may be 1.0 or less, 0.1 or less, 0.05 or less, or 0.01 or less, from the viewpoint of antagonistic action of n-6PUFA.

(n) Among the polyunsaturated fatty acids having 22 carbon atoms, in particular, the content of docosatetraenoic acid (C22:4, n-6, DTA) may be 2.0 area% or less, 1.5 area% or less, or 1.0 area% or less based on the total fatty acids. When the DTA content in an EPA-rich microbial oil is within this range, the EPA-rich microbial oil can achieve the same or superior effect considering the antagonistic action of n-6PUFA.

(n') In the EPA-rich microbial oil, the ratio of the content of the fatty acid DTA to that of EPA may be 1.0 or less, 0.1 or less, 0.05 or less, or 0.02 or less, considering the antagonistic action of n-6PUFA.

The EPA-rich microbial oil may have at least one of the fatty acid contents or ratios of fatty acids (a) to (n'), and may have at least one fatty acid content or ratio selected from the group consisting of (a) to (n'), from the viewpoint of containing EPA at a high concentration and effectively exhibiting the function of EPA.

The EPA-rich microbial oil may be a crude oil or a refined oil. The EPA-rich microbial oil is preferably a crude oil, from the viewpoint of, for example, high-purity EPA in the composition. As used herein, the term "crude oil of microbial oil" refers to a composition that is extracted from a biomass of a microorganism and is not subjected to a purification step described below. In the present specification, the term "extraction" means destroying the structure of the microorganism in the biomass and taking out the oil component in the microorganism. The extraction method and others will be described later. In the present specification, the term "refined oil of microorganisms" refers to a composition obtained by subjecting a crude oil to a de-gumming process, deacidification process, decoloration process using an activated clay or active carbon, washing process, deodorization process by steam distillation or the like, and then a crude oil purification step that removes substances other than the target substance, such as phospholipids and sterols.

In the EPA-rich microbial oil, each fatty acid may be present in any of the triglyceride, diglyceride and monoglyceride forms. The content of triglyceride in the EPA-rich microbial oil may be 70% by weight or greater, 75% by weight or greater, 80% by weight or greater, 90% by weight or greater, or 95% by weight or greater based on the total weight of the microorganism. When the triglyceride content in the EPA-rich microbial oil is 70% by weight or greater, the hygroscopicity of the microbial oil is not too low, and, for example good fluidity can be obtained. The upper limit of the triglyceride content in the EPA-rich microbial oil is not particularly limited, and is generally 99% by weight or less of the total weight of the EPA-rich microbial oil. In another aspect of the present disclosure, the triglyceride content in the EPA-rich microbial oil is 100% by weight based on the total weight of the EPA-rich microbial oil, that is, the EPA-rich microbial oil may be substantially free of other components than triglyceride. The triglyceride content in the EPA-rich microbial oil is measured according to AOCS Recommended Practice Cd 11c-93 (1997), SAMPLING AND ANALYSIS OF COMMERCIAL FATS AND OILS, (American Oil Chemists' Society).

The EPA-rich microbial oil may contain other components specific to a microbial oil, may exhibit a fatty acid composition specific to microorganisms, or may contain other components and exhibit a fatty acid composition specific to microorganisms. Examples of the other components specific to a microbial oil include phospholipids, free fatty acids, fatty acid esters, monoacylglycerols, diacylglycerols, triacylglycerols, sterols and sterol esters, carotenoids, xanthophylls, ubiquinones, hydrocarbons, isoprenoid-derived compounds, and compounds obtained by metabolizing any of them.

Since the EPA-rich microbial oil can be obtained without a chemical synthesis step, it may have a low organic solvent content. Organic solvent in the present specification means an organic solvent other than fatty acids, and means a hydrophobic or hydrophilic solvent having at least one carbon. Examples of the organic solvent include polar solvents, nonpolar solvents, water-miscible solvents, water-immiscible solvents, and combinations or at least two of these. Examples of the organic solvent include substituted or unsubstituted, saturated or unsaturated aliphatic hydrocarbons, aromatic hydrocarbons, alcohols, ethers, ketones, aldehydes, carboxylic acids, esters, nitriles, amides, and others. The organic solvent may be one of these types or a combination of at least two or more of these.

The total content of residual organic solvent in the EPA-rich microbial oil may be 5000 ppm or less, 3000 ppm or less, 2000 ppm or less, or 1000 ppm or less.

In the EPA-rich microbial oil, examples of the organic solvent having a low content include at least one selected from the group consisting of methanol, ethanol, acetone, and hexane. The content of these organic solvents may be each independently 500 ppm or less, 300 ppm or less, or 200 ppm or less. For example, each of the contents of methanol, ethanol, acetone, and hexane in the DPA-rich microbial oil may be 500 ppm or less, 300 ppm or less, or 200 ppm or less.

### Method for producing microbial oil

A method for producing a microbial oil according to an embodiment of the present disclosure includes preparing a microorganism having an ability to produce both eicosapentaenoic acid and docosahexaenoic acid, culturing the prepared microorganism in a medium containing glycerol under an environment aerated at 30 mL/min or more and 1500 mL/min or less, obtaining the EPA-rich microbial oil of the present disclosure from biomass obtained after culturing, and other steps as needed.

In the present specification, the step of preparing a microorganism having an ability to produce both eicosapentaenoic acid and docosahexaenoic acid may be simply referred to as a "microorganism preparation step". The step of culturing in a medium containing glycerol under an environment aerated at 30 mL/min or more and 1500 mL/min or less may be simply referred to as the "culturing step". The step of obtaining the EPA-rich microbial oil of the present disclosure from the biomass obtained after culturing may be simply referred to as the "microbial oil acquisition step".

In the microorganism preparation step, a microorganism having an ability to produce both EPA and DHA is prepared. The microorganism having an ability to produce both EPA and DHA is not particularly limited as long as it is a microorganism capable of producing EPA and DHA in the microorganism. The microorganisms can be culturable microorganisms and may be any culturable microorganisms after natural collection or after culturing. One species of these microorganisms may be used alone, or two or more species of them may be used in combination. The microorganism having an ability to produce both EPA and DHA according to an embodiment of the present disclosure may be a gene recombinant.

Examples of the applicable microorganisms may include at least one species selected from the group consisting of microorganisms belonging to fungi, microorganisms of green algae (Chlorophycea), microorganisms of Euglena algae (Euglenida), and microorganisms of SAR.

Examples of the fungi may include yeast and filamentous fungi. Examples of the yeast may include at least one species of microorganisms selected from the group consisting of microorganisms of the genus Yarrowia, microorganisms of the genus Pichia, microorganisms of the genus Saccharomyces, microorganisms of the genus Cryptococcus, and microorganisms of the genus Trichosporn. Examples of the filamentous fungi may include at least one species of microorganisms selected from the group consisting of microorganisms of the genus Mortierella, microorganisms of the genus Pythium, and microorganisms of the genus Phytophthora. Among them, microorganisms belonging to the genus Mortierella are more preferred. Examples of microorganisms belonging to the genus Mortierella may include Mortierella elongata, Mortierella exigua, Mortierella hygrophila, and Mortierella alpina.

Examples of microorganisms of green algae may include microorganisms belonging to the genus Botryococcus, Pseudochoricystis, Scenedesmus, and Desmodesmus.

Examples of microorganisms of Euglena algae may include microorganisms belonging to the family Euglenaceae. Examples of microorganisms belonging to the family Euglenaceae may include microorganisms belonging to the genus Euglena.

SAR means SPR in the classification system proposed by Adl et al. (Adl et. al., The Journal of Eukaryotic Microbiology. 59 (5): 429-493 (2012)), and specifically microorganisms of Stramenopiles, Alveolates, and Rhizaria are applicable. Among them, at least one species of microorganisms selected from the group consisting of microorganisms belonging to the Stramenopiles is preferred. Examples of the Stramenopiles may include at least one species of microorganisms selected from the group consisting of microorganisms of Bicosoecida, Labyrinthulea, Blastocystis, Actinophyida, Opalinata, Placidida, Oomycetes, Hyphochytriomycetes, Developayella, golden algae (Chrysophyceae), Eustigmatophyceae, Phaeothamniophyceae, Pinguiophyceae, Raphidophyceae, Synurophyceae, Xantexyophyceae, brown algae (Phaeophyceae), Schizocladiophyceae, Chrysomerophyceae, Dictyochophyceae, Bolidophiceae, Pelagophyceae, and diatoms (Bacillariophyceae).

Examples of the Alveolates may include microorganisms belonging to Ciliophora, Apicomplexa, and Dinoflagellate. Examples of the Dinoflagellate may include microorganisms belonging to the genus Crypthecodinium. Examples of the microorganisms belonging to the genus Crypthecodinium may include Crypthecodinium cohnii.

Among them, the microorganisms are preferably microorganisms belonging to Labyrinthulea, and among them, microorganisms belonging to the family Thraustochytrid are particularly preferred. The microorganisms belonging to the family Thraustochytrid are preferably, for example, microorganisms belonging to the genus Aplanochytrium, the genus Japonochytrium, the genus Labyrinthuloides, the genus Schizochytrium, the genus Thraustochytrium, the genus Ulkenia, the genus Aurantiochytrium, the genus Oblongichytrium, the genus Botryochytrium, the genus Parietichytrium, or the genus Sicyoidochytrium, and among them, at least one species of microorganisms selected from the group consisting of microorganisms of the genus Schizochytrium and Thraustochytrids are particularly preferred.

In one embodiment of the present disclosure, the microorganism capable of producing both EPA and DHA is, among the above-mentioned microorganisms, a microorganism that does not grow, or can produce more DHA than EPA, in a medium containing glucose. Glucose is a sugar that can be used as a carbon source or in other applications in the medium. Microorganisms that do not grow or can produce more DHA than EPA in a medium containing glucose exhibit a different behavior from the case of cultivation in the medium containing glucose in a specific culturing step described later, and as a result, can produce more EPA than DHA. As a result, from the viewpoint of the content of EPA in the microbial oil, a microorganism which has been considered to have an inferior EPA production efficiency can be used as a microorganism having a superior EPA production efficiency.

"Glucose-containing medium" means a medium in which the glucose content in the medium is from 1% by weight to 50% by weight, preferably from 10% by weight to 30% by weight based on the total weight of the medium. The medium will be described later. The phrase "does not grow in a medium containing glucose" means that the glucose concentration in the culture medium has not decreased compared to the concentration added to the medium after a certain period of time, for example, 24 hours, under the condition that the growth of the microorganism is possible. Therefore, "does not grow in a medium containing glucose" means that the microorganism cannot grow in a medium containing only glucose as carbon source. The method of confirming that "more DHA can be produced than EPA" can be based on the fatty acid content in the microbial oil obtained by cultivation.

In the culturing step, the prepared microorganism is cultured in a medium containing glycerol under an environment aerated at 30 mL/min or more and 1500 mL/min or less. The incubator used for culturing is not particularly limited, and may be appropriately selected from incubators capable of liquid cultivation from 1 mL to 10 L, or from 100 mL to 1 L scale depending on the type of microorganism. The incubator may have a stirring or osmotic function. The incubator may be, for example, a test tube, a flask, a Sakaguchi flask, a baffled flask, a tube-type incubator, a raceway-type incubator, or a panel-type incubator.

In the culturing step, culturing in an environment aerated at 30 mL/min or more and 1500 mL/min or less is equivalent not to a completely sealed condition but to an appropriately aerated condition, and can suppress excessive water evaporation. This aerated environment can be confirmed by closing the vent of the incubator with a plug and measuring the flow rate at 26 mm water column (255 Pa) with a float-type flowmeter. The aerated environment in the culturing step is required to be an environment in which an aeration amount of 30 mL/min or more and 1500 mL/min or less can be confirmed, and preferably an environment in which an aeration amount of 30 mL/min or more and 1000 mL or less, 30 mL/min or more and 500 mL or less, 30 mL/min or more and 200 mL or less, or 30 mL/min or more and 120 mL or less can be confirmed, from the viewpoints of EPA production efficiency and others.

This aerated environment can be achieved, for example, by closing the vent of the incubator with a plug capable of providing the aerated environment defined herein. A plug capable of providing this aerated environment can be selected by closing the vent of a 500 mL Erlenmeyer flask and measuring the flow rate at 26 mm water column with a float-type flowmeter. In the present specification, a plug capable of closing the vent of the incubator may be referred to as a "culture plug". This aerated environment is preferably equivalent to an environment in which the amount of water evaporated is 0.5 mL when the culture medium is left at 30°C for 10 days, from the viewpoint of suppressing excessive concentration of the culture medium. Examples of culture plug capable of providing an aerated environment in the culturing step include "Shin-Etsu-Silicosen T type" (T-42, Shin-Etsu Polymer Co., Ltd.) made of silicone rubber.

The culture medium applied in the microorganism culturing step contains glycerol. The glycerol in the culture medium usually acts as a carbon source, but may have other functions. For culture conditions and the like, the culture medium, culture conditions and others that have been typically applied to the microorganism may be applied as they are.

The content of glycerol in the culture medium may be from 0.1% to 50% by weight, preferably from 1% to 30% by weight, from the viewpoint of maintaining or promoting the growth of the microorganism. As the microorganism grows, the carbon source may be added to the culture medium in an appropriate amount an arbitrary number of times. When the carbon source is added, the amount of carbon source added at one time may be appropriately set from the viewpoint of maintaining or promoting the growth of the microorganism.

The culture medium may contain glycerol as a main component, and further carbon sources such as fructose, xylose, sucrose, maltose, starch, soluble starch, molasses, glucose, trehalose, mannitol, and other compounds available as carbon sources, to the extent that the purpose of the method for producing an EPA-rich microbial oil according to the present disclosure is not impaired

When these carbon sources are used in combination with glycerol, the total content of these carbon sources in the culture medium is 0.01 or more based on the content of glycerol, and may be 0.5 or less, and preferably 0.3 or less. The culture medium containing glycerol may contain glucose at a glucose concentration at which the microorganism grows and the content of eicosapentaenoic acid in the microbial oil is higher than the content of docosahexaenoic acid.

Generally, the culture is started by inoculating a liquid medium or a solid medium with spores, hyphae, or a pre-culture liquid obtained by culturing a microorganism in advance. Examples of the medium include media including appropriate combinations of yeast extract, malt extract, meat extract, fish meat extract, corn steep liquor, peptone, polypeptone, soybean powder, defatted soybean powder, casamino acid, urea, sodium glutamate, ammonium acetate, ammonium sulfate, ammonium nitrate, ammonium chloride, and sodium nitrate as nitrogen sources; sodium chloride, potassium chloride, magnesium chloride, magnesium sulfate, calcium chloride, potassium dihydrogen phosphate, dipotassium hydrogen phosphate as inorganic salts; vitamins, and other necessary components. A culture medium containing sodium chloride, potassium chloride, magnesium chloride, magnesium sulfate, calcium chloride, and others can be applied to cultivation of marine microorganisms.

The nitrogen source in the culture medium may be from 0.01% to 10% by weight, preferably from 0.1% to 4% by weight, from the viewpoint of maintaining or enhancing the growth of the microorganism. As the microorganism grows, an appropriate amount of nitrogen source may be added to the culture medium, an arbitrary number of times. When the nitrogen source is added, the amount of nitrogen source added at one time may be appropriately set from the viewpoint of maintaining or enhancing the growth of the microorganism.

As the culture medium, for example, glycerol/yeast extract agar medium (GlyY medium) may be used in terms of the growth possibility of the microorganisms that can produce a wide variety of EPA-rich microbial oils. The aqueous medium as the base material of the liquid medium is basically water, and may be distilled water or purified water. After the medium is prepared, the pH is adjusted within the range from 3.0 to 9.0, and the medium is sterilized by an autoclave or the like before use.

The pH of the culture medium may be appropriately selected from the viewpoint of the growth of microorganisms, the synthesis of fatty acids, and the maintenance or enhancement of the ability to produce EPA, and is, for example, from 3.0 to 9.5, preferably from 3.5 to 9.5, and even more preferably from 4.5 to 9.0. The pH of the culture medium may be appropriately controlled by a pH adjuster such as sodium hydroxide or sulfuric acid when the components in the culture medium are changed by being metabolized by the microorganism.

The culture temperature may be, for example, 40°C or lower, 35°C or lower, 30°C or lower, 28°C or lower, or 25°C or lower from the viewpoint of EPA production efficiency, while may be 8°C or higher, 10°C or higher, or 15°C or higher from the viewpoint of development of the microorganism. The upper limit or the lower limit of the culture temperature may be any combination, and may be, for example, from 8°C to 40°C, from 8°C to 28°C, from 10°C to 40°C, from 10°C to 25°C, or from 15°C to 25°C. When culturing in such a temperature range, the concentration of EPA in the EPA-rich microbial oil can be increased.

The culturing period in the culturing step varies depending on, for example, the type of microorganism, development state, and efficiency of fatty acid accumulation, and is not particularly limited, but may be terminated when the amount of dry biomass per liter of the culture medium is 1 to 100 g. The culturing period in the culturing step may be usually from 1 to 30 days, or from 3 to 10 days.

In the microbial oil acquisition step, the EPA-rich microbial oil of the present disclosure is obtained from the biomass obtained by culturing. The microbial oil acquisition step may include a harvest step of harvesting the biomass after the culture period and an extraction step of extracting microbial oil from the harvested biomass.

In the recovery step, the biomass after culturing can be recovered from the culture medium by a method known in the art depending on the type of organism and the type of medium. By the recovery step, biomass containing EPA-rich microbial oil is obtained. When cultivation is performed by liquid culture, for example, the recovery treatment can be generally performed after the cultivation is completed using solid-liquid separation means such as centrifugation and filtration from the culture medium. When cultivation is performed in a solid medium, the solid medium and the biomass can be crushed with a homogenizer or the like without separating the biomass and the medium, and the obtained crushed product can be subjected to the subsequent extraction step.

In the extraction step, oil is extracted from the biomass after the harvest step to obtain extracted oil. For the extraction treatment, a usual extraction method using an organic solvent can be applied. When the harvested biomass is dry biomass, the extraction treatment may be extraction with supercritical carbon dioxide or extraction treatment with an organic solvent under a nitrogen gas stream. Examples of organic solvents used for the extraction treatment include ethers such as dimethylether and diethylether; hydrocarbons having at most 10 carbon atoms such as petroleum ether, hexane, and heptane; alcohols such as methanol and ethanol; chloroform; dichloromethane; fatty acids such as octanoic acid or alkyl esters thereof; and oils such as vegetable oil. As the extraction treatment, alternate extraction with methanol and petroleum ether or extraction using a single-layer solvent of chloroform-methanol-water may be applied. In this case, a better result of extraction treatment can be obtained. An extracted oil can be obtained by distilling off the organic solvent from the extract under reduced pressure. Hexane is generally used as an organic solvent when collecting triacylglycerol or extracting an extracted oil from a microorganism. Crude oil refers to the extracted oil obtained immediately after the extraction process, as described above.

When wet cells are used in the extraction treatment, the wet cells may be squeezed, or a solvent may be used. Examples of the usable solvent include a solvent miscible with water such as methanol and ethanol, or a mixed solvent of a solvent miscible with water and water and/or another solvent. The remainder of the procedure is similar to that described above.

When the EPA-rich microbial oil is a refined oil, the method for producing the EPA-rich microbial oil may include an extraction step, followed by refinement treatment including subjecting the obtained extracted oil, or crude oil to a degumming step, deoxidation step, decolorization step, and deodorization step. By this purification treatment, an EPA-rich microorganism as a refined oil can be obtained. By performing the purification step on the extracted oil derived from microbial biomass, a triacylglycerol concentrate having a concentration of triacylglycerol higher than that of the crude oil is mainly obtained.

In the purification step, the crude oil is degummed, deoxidized, decolorized, and deodorized by methods known to those skilled in the art by the method used for purifying vegetable oil, fish oil and others. The degumming treatment is performed, for example, by washing with water. The deoxidation treatment is performed, for example, by distillation treatment. The decolorization treatment is performed by, for example, decolorization treatment using activated clay, activated carbon, silica gel, or the like. The deodorizing treatment is performed by, for example, steam distillation.

The EPA-rich microbial oil obtained by the method for producing an EPA-rich microbial oil may be subjected to a processing step in which the crude oil or the refined oil is subjected to processing such as hydrolysis and alkyl esterification after the extraction step in the microbial oil acquisition step, and optionally after the extraction step and the purification step. By the processing step, for example, a composition containing free EPA derived from EPA-rich microbial oil, and a composition containing lower alkyl ester EPA derived from EPA-rich microbial oil, can be obtained. In particular, when the purification step and the processing step are carried out in this order after the extraction step, a composition containing free fatty acids derived from the triacylglycerol concentrate obtained in the purification step, and a composition containing a fatty acid lower alkyl ester, can be obtained. The alkyl esterification treatment and the hydrolysis treatment in the processing step can be performed under conditions known to those skilled in the art.

A lower alcohol is used for the alkyl esterification treatment. Examples of the lower alcohol may include alcohols having from 1 to 3 carbon atoms, such as methanol, ethanol and propanol. For example, fatty acid methyl ester is obtained by treating the extracted oil with, on a volume basis, from 5% to 10% of anhydrous methanol-hydrochloric acid, or from 10% to 50% of BF₃-methanol at room temperature for 1 to 24 hours. The ethyl ester of fatty acid can be obtained by treating the extracted oil with from 1% to 20% sulfuric acid ethanol or the like at 25°C to 100°C for 15 minutes to 60 minutes. The methyl esters or ethyl esters may be extracted from the reaction liquid using an organic solvent such as hexane, diethylether, or ethyl acetate. The extract liquid is dried using anhydrous sodium sulfate or the like, and then the organic solvent is removed by distillation to obtain a composition mainly composed of fatty acid alkyl esters.

The hydrolysis treatment may be carried out under conditions known to those skilled in the art. For example, the extracted oil is subjected to alkali decomposition treatment at room temperature for from 2 hours to 3 hours with 5% sodium hydroxide, and then a composition containing free fatty acid is obtained from the obtained decomposition solution by a method commonly used for extraction or purification of fatty acids.

Both alkyl esterification and hydrolysis may be performed to obtain a free fatty acid. By continuously performing the alkyl esterification treatment and the hydrolysis treatment, a higher purity free fatty acid can be obtained. In order to obtain a free fatty acid from a fatty acid alkyl ester, for example, hydrolysis with an alkali catalyst is followed by extraction treatment with an organic solvent such as ether or ethyl acetate.

After the above-mentioned purification step or after processing, the EPA-rich microbial oil or the composition derived therefrom may be subjected to a concentration treatment for the purpose of reducing the concentration of compounds containing fatty acids other than EPA as constituent fatty acids, and increasing the concentration of EPA. For the concentration treatment, distillation, rectification, column chromatography, low temperature crystallization method, urea inclusion method, liquid-current countercurrent distribution chromatography, and others may be applied singly or in combination of two or more. A combination of distillation or rectification, and column chromatography or liquid-liquid countercurrent distribution chromatography, is preferably used. Reverse phase distribution type column chromatography is preferred as the column chromatography. When the step of concentrating or isolating EPA is performed, the content of EPA in the fatty acids that can be finally contained in the EPA-rich microbial oil can be increased, and the content of fatty acids other than EPA in the fatty acids can be reduced.

For example, when rectification is used, the rectification step is preferably carried out under the conditions that the pressure at the top of the distillation column is reduced to 10 mmHg (1333 Pa) or less and the bottom temperature is from 165°C to 210°C, preferably from 170°C to 195°C, thereby suppressing the denaturation of the fatty acid by heat and increasing the rectification efficiency. The pressure of the upper part of the distillation column is preferably as low as possible, and more preferably less than or equal to 0.1 mmHg (13.33 Pa). The temperature of the upper part of the column is not particularly limited, and may be, for example, 160°C or lower. Through the rectification step, a composition with a higher content of EPA can be obtained.

The reverse phase column chromatography may be reverse phase column chromatography that is known in the art, and high-performance liquid chromatography (HPLC) using a base material modified with octadecylsilyl groups (ODS) as a stationary phase is particularly preferable. By performing such a concentration treatment, an EPA concentrated oil having an EPA concentration of, for example, from 90% to 98% by weight, from 95% to 98% by weight, from 96% to 98% by weight, or from 97% to 98% by weight of the total weight of fatty acids in the oil can be obtained.

Another aspect of the invention includes a method of controlling the fatty acid content of a microbial oil. The method for controlling fatty acid content ratio of the microbial oil according to the present disclosure includes preparing a microorganism that does not grow, or can produce a microbial oil containing more docosahexaenoic acid than eicosapentaenoic acid, in a medium containing glucose, culturing the prepared microorganism in a culture medium containing glycerol under an environment aerated at 30 mL/min or more and 1500 mL/min or less, obtaining a microbial oil containing more eicosapentaenoic acid than docosahexaenoic acid from biomass obtained after culturing, and other steps as necessary.

According to the present control method, the fatty acid composition of the microbial oil produced by a specific microorganism can be easily controlled. This can expand the selection range of microbial species that can be used to efficiently obtain an EPA-containing composition such as an EPA-rich microbial oil. In the present embodiment, "control of the fatty acid composition of microbial oil" means changing the fatty acid composition of microbial oil that can be produced by a microorganism according to the conditions for culturing the microorganism. An embodiment in which the microorganism that does not grow in the present embodiment changes to a state capable of producing a microbial oil having a predetermined fatty acid composition is also included in one embodiment of "control of the fatty acid composition of microbial oil".

In the method for controlling the content ratio of fatty acid according to the present disclosure, a microorganism is prepared that does not grow, or can produce a microbial oil that contain more docosahexaenoic acid than eicosapentaenoic acid, in a medium containing glucose. The "microorganism that does not grow, or can produce a microbial oil containing more docosahexaenoic acid than eicosapentaenoic acid, in a medium containing glucose" used in this step corresponds to the microorganism described in the "microorganism preparation step" in the method for producing a microbial oil according to the other aspect of the present disclosure.

Next, the prepared microorganism is cultured in a culture medium containing glycerol in an environment aerated at 30 mL/min or more and 1500 mL/min or less, preferably 30 mL/min or more and 120 mL/min or less. The matters described in the "culturing step" in the method for producing a microbial oil according to the other aspect of the present disclosure can be applied to the matters relating to this step.

Next, a microbial oil containing more eicosapentaenoic acid than docosahexaenoic acid is obtained from the biomass obtained after culturing. The microbial oil obtained here is a microbial oil having a content ratio of DHA and EPA different from the microbial oil obtained when culturing in a culture medium containing glucose. The matters described in the "microbial oil acquisition step" in the method for producing a microbial oil according to the other aspect of the present disclosure can be applied to the matters related to this step.

The EPA-rich microbial oil can be applied in applications in which various functions of EPA are required to be ingested efficiently. Examples of products containing the EPA-rich microbial oil may include foods, supplements, pharmaceuticals, cosmetics, and feeds. The EPA-rich microbial oil may be used in methods of producing these products. Examples of the use of the EPA-rich microbial oil include foods, supplements, pharmaceuticals, cosmetics, and feeds that contain particularly EPA as an active ingredient and are expected to prevent lifestyle-related diseases such as arteriosclerosis, cerebral infarction, myocardial infarction, thrombosis, and hyperlipidemia, improve metabolic syndrome, and have anti-allergy, anti-inflammatory, anti-cancer, and dementia effects. Examples of the medicament include external medicines for skin, and oral preparations.

The usage form of the EPA-rich microbial oil is not particularly limited, and it may be used as a liquid component or a solid component. For example, when an EPA-rich microbial oil is applied to products such as foods, supplements, pharmaceuticals, cosmetics, and feeds, the EPA-rich microbial oil itself may be commercialized by combining it with other optional components, or may be commercialized by performing any additional treatment before or after combining with other optional components. Examples of such additional processing may include powdering, pelletizing, encapsulating, and tableting.

When the EPA-rich microbial oil is used as a medicament, the medicament contains the EPA-rich microbial oil and a pharmaceutically acceptable carrier and, as necessary, other components. The dosage form may be any form as long as oral or parenteral administration is conveniently carried out. Examples of the dosage form include injections, transfusions, powders, granules, tablets, capsules, enteric coated tablets, troches, peroral liquid preparations, suspensions, emulsions, syrups, liquids for external use, fomentations, nasal preparations, eardrops, eye drops, inhalants, ointments, lotions, and suppositories. These may be used individually or in combination depending on the symptoms.

By usual methods, these various types of preparations, according to a purpose, may be formulated by adding, to the principal agent, previously known adjuvants commonly used in the field of drug preparation technology, as exemplified by excipients, binders, preservatives, stabilizers, disintegrants, lubricants, flavoring agents, or the like. Furthermore, in the case of oral administration for an adult, typically, the dosage for administration can be appropriately adjusted in a range from 0.01 mg to 10 g, preferably from 0.1 mg to 2 g, and more preferably from 1 mg to 200 mg, per day as the total amount of the LC-PUFA as a structured lipid. In the case of parenteral administration, the dosage for administration can be appropriately adjusted in a range from 0.001 mg to 1 g, preferably from 0.01 mg to 200 mg, and more preferably from 0.1 mg to 100 mg, per day as the total amount of the EPA as a structured lipid. However, these dosages differ depending on purpose of the administration and conditions of the person subjected to the administration (sex, age, weight, and the like).

In addition, in this specification, the features of each invention described in embodiments related to each aspect of the invention may be combined as desired to form new embodiments, and it is to be understood that such new embodiments may be included in each of the aspects of the present invention.

In the present specification, unless otherwise noted, when a numerical range that only specifies one or a plurality of upper limit values and a numerical range that only specifies one or a plurality of lower limit values are described for an identical subject, an embodiment of the present invention includes a numerical range having a combination of any upper limit value that is chosen from the one or the plurality of the upper limit values and any lower limit value that is chosen from the one or the plurality of the lower limit values.

### EXAMPLES

Hereinafter, the present disclosure will be described in detail with reference to examples. However, the present invention is not limited in any manner by these examples. Unless otherwise noted, "parts" or "%" are on a mass basis.

Unless otherwise specified, the term "cell" or "microbial cell" in the following Examples means an aggregate of cells or microbial cells, and corresponds to biomass in the present specification.

### Example 1

"SEK 704 AB973560 strain" (Aquat. Microb. Ecol., 2015, Vol. 74, pp. 187-204), which is a related species of Thraustochytrium sp. AR2-1 AB810959, Family Thraustochytriidae, was obtained from Biotechnology Center, National Institute of Technology and Evaluation (Unidentified Thraustochytrid 5, NBRC110858, hereinafter referred to as microorganism A).

Microorganism A was suspended in 50% artificial seawater described below, and then 0.1 ml of the microbial suspension was inoculated into a sterile 500-mL pleated shake flask containing 100 mL of liquid culture medium for Thraustochytrids of the following composition (pH 6.8). 50% artificial seawater was prepared by mixing the Herbst formulation shown in Table 4 and purified water at a volume ratio of 1:1.

After inoculating the microorganisms the mouth of the flask was plugged with a silicone rubber culture plug "Shin-Etsu Silicosen T Type" (Shin-Etsu Polymer Co., Ltd.). "Shin-Etsu Silicosen T Type" exhibits an aeration amount of 30 to 120 mL/min when the vent of a 500 mL Erlenmeyer flask is plugged with the plug, and the flow rate at 26 mm water column is measured with a float-type flowmeter, and exhibits a water evaporation amount of 0.5 mL or less at 30°C for 10 days when 5 mL of water is injected into a medium test tube, the sample is left to stand with the plug attached, and the increase or decrease in weight due to water evaporation is measured over time. Bio-Shaker BR-300LF (Taitec Corporation) was used as a culture incubator, and this flask was shaken for 3 days at 28°C and 130 rpm.

**Table 1**

| Liquid medium for Thraustochytrid (1 liter) | |
|---|---|
| Glycerol | 30 g |
| Yeast extract | 10 g |
| NaCl | 15 g |
| KCl | 0.35 g |
| MgCl₂•6H₂O | 5.4 g |
| MgSO₄•7H₂O | 2.7 g |
| CaCl₂•2H₂O | 0.5 g |
| Vitamin solution^{∗} | 1.0 mL |
| Metal salt solution^{∗∗} | 1.0 mL |
| Deionized water | Remainder |

**Table 2**

| Vitamin solution (1 liter) | |
|---|---|
| Thiamine hydrochloride | 9.75 g |
| Calcium pantothenate | 3.33 g |
| Cyanocobalamin | 0.16 g |
| Biotin | 3.58 g |
| Deionized water | Remainder |

**Table 3**

| Metal salt solution (1 liter) | |
|---|---|
| Citric acid | 46.8 g |
| FeSO₄•7H₂O | 10.3 g |
| MnCl₂•4H₂O | 3.1 g |
| ZnSO₄•7H₂O | 9.3 g |
| CoCl₂•6H₂O | 0.04 g |
| NaMoO₄•2H₂O | 0.04 g |
| CuSO₄•5H₂O | 2.07 g |
| NiSO₄•6H₂O | 2.07 g |
| Deionized water | Remainder |

**Table 4**

| Herbst formulation (1 liter) | |
|---|---|
| NaCl | 30 g |
| KCl | 0.7 g |
| MgCl₂•6H₂O | 10.8 g |
| MgSO₂•7H₂O | 5.4 g |
| CaCl₂•2H₂O | 1.0 g |
| Deionized water | Remainder |

20 ml of the cultured cell suspension obtained by culturing in the shake flask was harvested in a sterile 50 ml test tube and centrifuged to harvest cells. The harvested cells were washed twice with artificial seawater. Then, the cells were frozen at -80°C and then subjected to a lyophilizer (VA-140S available from TAITEC Corporation) to obtain lyophilized microorganism cells A. From the obtained lyophilized cells, total lipids, that is, microbial oil A (T type) was obtained as follows, and then the obtained microbial oil A (T type) was further converted into fatty acid methyl ester.

According to the method of Folch et al. (J. Biological and Chemistry 226: 497-509 (1957)), total lipids were extracted from the lyophilized cells with chloroform:methanol (2:1, v/v). The total lipids thus obtained were methyl-esterified to obtain a fatty acid methyl ester (FAME). The FAME thus obtained was subjected to FAME analysis by gas chromatography. The conditions for the gas chromatography were set as follows.
- Column: DB-WAX 0.530 mm × 30 m, membrane thickness: 1.00 µm (Agilent Technologies);
- Carrier gas conditions: helium 1.0 ml/min, separation ratio 100:1
- Column temperature conditions: 5 minutes at 140°C, temperature rise up to 240°C at 4°C/min, 10 minutes at 240°C, detection by FID
- Detector temperature: 260°C
- Inlet temperature: 250°C
- Injection volume: 1 µL

As a result, microbial oil A (T type) contained 40.3 area% of EPA based on the total fatty acids and 21.3 area% of DHA based on the total fatty acids. The ratio of the content of EPA to the content of DHA was 1.89.

As a comparative control, microorganism A was cultured under the same conditions as described above, except that "Shin-Etsu Silicosen C Type" (Shin-Etsu Polymer Co., Ltd.) was used in place of the silicone rubber culture cap "Shin-Etsu Silicosen T Type" (Shin-Etsu Polymer Co., Ltd.). "Shin-Etsu Silicosen C Type" exhibits an aeration volume of 2000 to 4500 mL/min when the vent of a 500 mL Erlenmeyer flask is plugged with the cap, and the flow rate at the time of 26 mm water column is measured with a float-type flowmeter, and exhibits a water evaporation amount of more than 0.5 mL at 30°C for 10 days when 5 mL of water is injected into a medium test tube, the sample is left to stand with the cap attached, and the increase or decrease in weight due to water evaporation is measured over time. After culturing, microbial oil A (C type) was obtained, and microbial oil A (C type) was converted to FAME in the same manner as in Example 1, and then subjected to fatty acid analysis.

As a result, microbial oil A (C type) obtained from microorganism A contained 11.6 area% of EPA based on the total fatty acids and 52.1 area% of DHA based on the total fatty acids. The ratio of the content of EPA to the content of DHA was 0.22.

As described above, microorganism A was found to produce a microbial oil (T type) containing more EPA than DHA when cultured in a culture medium containing glycerol instead of glucose in an aerated environment with an aeration amount of 30 to 120 mL/min.
As a result, a microbial oil containing EPA at a high content of 38 area% or more and having a ratio of the content of EPA to the content of DHA of 1.7 or more was obtained.

### Example 2

Microbial oils A(Gly) to G(Gly) were obtained by culturing the microorganisms in the same manner as in Example 1, except that microorganism A used in Example 1 and microorganisms B to G listed below were used, and the number of days for cultivation was set to 5 days.
Microorganism A: Unidentified Thraustochytrid5 (NBRC110858)
Microorganism B: Schizochytrium sp. (NBRC102615)
Microorganism C: Thraustochytrium aureum (ATCC34304)
Microorganism D: Schizochytrium aggregatum (ATCC28209)
Microorganism E: Unidentified Thraustochytrid1 (NBRC110846)
Microorganism F: Thraustochytrium roseum (ATCC28210)
Microorganism G: Thraustochytrium striatum (ATCC24473)

The fatty acid compositions of the microbial oils A(Gly) to G(Gly) are shown in Tables 5 and 6.

The triglyceride contents of the microbial oils A(Gly) to G(Gly) were all 70% by weight or more.

As shown in Table 5 and Table 6, all of the microbial oils A(Gly) to G(Gly) are microbial oils containing 38 area% or more of EPA and more EPA than DHA, and are microbial oils having the fatty acid composition ratios shown in Table 5 and Table 6. From this, the microbial oils A(Gly) to G(Gly) contain EPA at a high concentration and have the advantage that they can effectively exert the function of EPA.

As comparative controls, microbial oils A(Glu) to G(Glu) were obtained in the same manner as the cultivation to obtain the microbial oils A(Gly) to G(Gly), except that microorganisms A to G were used in a liquid medium for Thraustochytrids (pH 6.8) having the same components except that it contained the same amount of glucose instead of glycerol.

The results are shown in Tables 7 and 8. In the table, "-" means that there is no corresponding microbial oil, or that there is no corresponding numerical value because the content of the fatty acid to be calculated is "0".

Table 7 and Table 8 indicate that microorganisms B, C, E, and F produce microbial oil B (Glu), microbial oil C (Glu), microbial oil E (Glu), and microbial oil F (Glu) that all contain more DHA than EPA in a culture medium containing glucose as a carbon source. It was confirmed that microorganisms A, D, and G did not grow in the culture medium containing glucose as a carbon source.

Further, all of microorganisms A to G were microorganisms that did not grow in a culture medium containing glucose as a carbon source, or produced microbial oil containing more DHA than EPA. As described above, the microbial oils A(Gly) to G(Gly) contained more EPA than DHA. As described above, it was found that a microbial oil having different fatty acid content ratios can be obtained by selecting predetermined culture conditions. As a result, it was found that the selection range of microbial species that can be used to efficiently obtain the EPA-containing composition can be easily expanded.

By using this microbial oil, EPA can be obtained more efficiently, and various products containing EPA can be efficiently provided.

**Table 5**

| | Microbial oil | | | | | | |
|---|---|---|---|---|---|---|---|
| | A (Gly) | B (Gly) | C (Gly) | D (Gly) | E (Gly) | F (Gly) | G (Gly) |
| Microbial species | A | B | C | D | E | F | G |
| C14:0 | 0.6 | 0.7 | 0.8 | 0.8 | 0.7 | 0.8 | 0.8 |
| C16:0 | 12.0 | 14.2 | 16.1 | 16.8 | 14.9 | 17.8 | 14.5 |
| C16:1 | 0.0 | 0.0 | 0.0 | 0.0 | 0.0 | 0.0 | 0.0 |
| C17:0 | 0.1 | 0.1 | 0.1 | 0.1 | 0.1 | 0.1 | 0.0 |
| C18:0 | 0.4 | 0.5 | 0.5 | 0.5 | 0.5 | 0.5 | 0.5 |
| C18:1 n-9 | 0.0 | 0.0 | 0.0 | 0.0 | 0.0 | 0.0 | 0.0 |
| C18:2 n-6 | 0.0 | 0.0 | 0.1 | 0.2 | 0.0 | 0.2 | 0.2 |
| C18:3 n-6 | 0.2 | 0.2 | 0.2 | 0.3 | 0.2 | 0.2 | 0.3 |
| C20:3 n-6 (DGLA) | 0.0 | 0.0 | 0.0 | 0.0 | 0.0 | 0.0 | 0.0 |
| C20:4 n-6 (ARA) | 2.1 | 2.0 | 1.9 | 1.8 | 1.9 | 1.7 | 1.9 |
| C21:0 | 0.7 | 0.6 | 0.5 | 0.6 | 0.5 | 0.5 | 0.6 |
| C20:4 n-3 (ETA) | 1.6 | 1.5 | 1.4 | 1.4 | 1.3 | 1.3 | 1.4 |
| C20:5 n-3 (EPA) | 55.1 | 52.8 | 50.8 | 51.0 | 50.3 | 49.3 | 53.4 |
| C22:4 n-6 (DTA) | 0.7 | 0.6 | 0.5 | 0.5 | 0.5 | 0.5 | 0.5 |
| C22:5 n-6 (n-6 DPA) | 0.0 | 0.0 | 0.0 | 0.0 | 0.0 | 0.0 | 0.0 |
| C22:5 n-3 (n-3 DPA) | 9.3 | 9.7 | 10.4 | 10.6 | 10.8 | 11.2 | 10.2 |
| C22:6 n-3 (DHA) | 10.3 | 10.1 | 11.1 | 10.4 | 11.4 | 10.6 | 10.7 |
| Others | 6.5 | 6.4 | 5.1 | 4.4 | 6.3 | 4.7 | 4.6 |

**Table 6**

| | Microbial oil | | | | | | |
|---|---|---|---|---|---|---|---|
| | A (Gly) | B (Gly) | C (Gly) | D (Gly) | E (Gly) | F (Gly) | G (Gly) |
| Microbial species | A | B | C | D | E | F | G |
| EPA/DHA | 5.3 | 5.2 | 4.6 | 4.9 | 4.4 | 4.7 | 5.0 |
| C18/C20 | 0.010 | 0.012 | 0.015 | 0.018 | 0.013 | 0.017 | 0.018 |
| C18/C22 | 0.030 | 0.034 | 0.036 | 0.046 | 0.031 | 0.040 | 0.047 |
| Total saturated fatty acids | 13.7 | 16.0 | 17.8 | 18.7 | 16.6 | 19.6 | 16.4 |
| Saturated/EPA | 0.25 | 0.30 | 0.35 | 0.37 | 0.33 | 0.40 | 0.31 |
| ARA/EPA | 0.038 | 0.038 | 0.037 | 0.035 | 0.038 | 0.034 | 0.036 |
| ETA/EPA | 0.03 | 0.03 | 0.03 | 0.03 | 0.03 | 0.03 | 0.03 |
| Total n-3 C22 PUFAs | 19.6 | 19.8 | 21.5 | 21.1 | 22.3 | 21.8 | 20.8 |
| Total n-3 C22 PUFAs/EPAs | 0.5 | 0.5 | 0.5 | 0.5 | 0.6 | 0.5 | 0.5 |
| n-3 DPA/EPA | 5.94 | 5.44 | 4.90 | 4.79 | 4.64 | 4.39 | 5.24 |
| n-6 DPA/EPA | 0.000 | 0.000 | 0.000 | 0.000 | 0.000 | 0.000 | 0.000 |
| n-6 DPA/n-3 DPA | 0.000 | 0.000 | 0.000 | 0.000 | 0.000 | 0.000 | 0.000 |
| Total unsaturated fatty acids having 18 carbon atoms | 0.2 | 0.2 | 0.3 | 0.5 | 0.2 | 0.4 | 0.5 |
| (Total unsaturated fatty acids having 18 carbon atoms)/EPA | 0.004 | 0.004 | 0.006 | 0.010 | 0.004 | 0.008 | 0.009 |
| C21:0/EPA | 0.013 | 0.011 | 0.010 | 0.012 | 0.010 | 0.010 | 0.011 |
| DGLA/EPA | 0.000 | 0.000 | 0.000 | 0.000 | 0.000 | 0.000 | 0.000 |
| DTA/EPA | 0.013 | 0.011 | 0.010 | 0.010 | 0.010 | 0.010 | 0.009 |

**Table 7**

| | Microbial oil | | | | | | |
|---|---|---|---|---|---|---|---|
| | A (Glu) | B (Glu) | C (Glu) | D (Glu) | E (Glu) | F (Glu) | G (Glu) |
| Microbial species | A | B | C | D | E | F | G |
| C14: 0 | - | 3.0 | 1.3 | - | 1.1 | 0.7 | - |
| C16:0 | - | 27.8 | 24.5 | - | 15.2 | 19.4 | - |
| C16:1 | - | 0.6 | 0.0 | - | 0.1 | 0.1 | - |
| C17:0 | - | 0.2 | 0.1 | - | 0.0 | 0.0 | - |
| C18:0 | - | 19.2 | 15.5 | - | 0.9 | 4.4 | - |
| C18:1 n-9 | - | 9.5 | 1.2 | - | 1.0 | 1.5 | - |
| C18:2 n-6 | - | 2.4 | 1.0 | - | 0.1 | 0.9 | - |
| C18:3 n-6 | - | 0.3 | 0.0 | - | 0.0 | 0.9 | - |
| C20:3 n-6 (DGLA) | - | 0.8 | 0.0 | - | 0.0 | 0.5 | - |
| C20:4 n-6 (ARA) | - | 9.6 | 1.5 | - | 2.9 | 7.9 | - |
| C21:0 | - | 0.0 | 0.0 | - | 0.0 | 0.0 | - |
| C20:4 n-3 (ETA) | - | 0.0 | 0.0 | - | 0.0 | 0.0 | - |
| C20:5 n-3 (EPA) | - | 3.5 | 3.0 | - | 17.5 | 5.6 | - |
| C22:4 n-6 (DTA) | - | 0.7 | 0.0 | - | 0.0 | 1.3 | - |
| C22:5 n-6 (n-6 DPA) | - | 7.3 | 7.1 | - | 4.8 | 12.0 | - |
| C22:5 n-3 (n-3 DPA) | - | 0.0 | 0.0 | - | 0.0 | 0.0 | - |
| C22:6 n-3 (DHA) | - | 10.9 | 41.1 | - | 49.3 | 39.8 | - |
| Others | - | 3.7 | 3.2 | - | 6.6 | 4.5 | - |

**Table 8**

| | Microbial oil | | | | | | |
|---|---|---|---|---|---|---|---|
| | A (Glu) | B (Glu) | C (Glu) | D (Glu) | E (Glu) | F (Glu) | G (Glu) |
| Microbial species | A | B | C | D | E | F | G |
| EPA/DHA | - | 0.3 | 0.1 | - | 0.4 | 0.1 | - |
| C18/C20 | - | 2.259 | 3.933 | - | 0.098 | 0.550 | - |
| C18/C22 | - | 1.661 | 0.367 | - | 0.037 | 0.145 | - |
| Total saturated fatty acids | - | 50.0 | 41.3 | - | 17.2 | 24.5 | - |
| Saturated/EPA | - | 14.3 | 13.8 | - | 1.0 | 4.4 | - |
| ARA/EPA | - | 2.743 | 0.500 | - | 0.166 | 1.411 | - |
| ETA/EPA | - | 0.00 | 0.00 | - | 0.00 | 0.00 | - |
| Total n-3 C22 PUFAs | - | 18.2 | 48.2 | - | 54.1 | 51.8 | - |
| Total n-3 C22 PUFAs/EPAs | - | 4.2 | 14.8 | - | 3.2 | 7.9 | - |
| n-3 DPA/EPA | - | - | - | - | - | - | - |
| n-6 DPA/EPA | - | 2.1 | 2.4 | - | 0.3 | 2.1 | - |
| n-6 DPA/n-3 DPA | - | - | - | - | - | - | - |
| Total unsaturated fatty acids having 18 carbon atoms | - | 12.2 | 2.2 | - | 1.1 | 3.3 | - |
| (Total unsaturated fatty acids having 18 carbon atoms)/EPA | - | 3.486 | 0.733 | - | 0.063 | 0.589 | - |
| C21:0/EPA | - | 0.000 | 0.000 | - | 0.000 | 0.000 | - |
| DGLA/EPA | - | 0.229 | 0.000 | - | 0.000 | 0.089 | - |
| DTA/EPA | - | 0.200 | 0.000 | - | 0.000 | 0.232 | - |

The following numbered paragraphs (paras) contain further statements of various aspects of the present invention:-
1. A microbial oil comprising eicosapentaenoic acid (EPA) and docosahexaenoic acid (DHA), the content of eicosapentaenoic acid being 38 area% or greater based on the total fatty acids, and the microbial oil having at least one fatty acid ratio selected from (a) to (c):
   (a) a ratio of the content of EPA to the content of DHA of 1.7 or higher;
   (b) a ratio of a content of fatty acids having 18 carbon atoms to the content of fatty acids having 20 carbon atoms of 0.3 or less;
   (c) a ratio of a content of fatty acids having 18 carbon atoms to a content of fatty acids having 22 carbon atoms of 1.5 or less.
2. Microbial oil according to para 1, wherein a content of triglyceride is 70% by weight or more based on a total weight of the microbial oil.
3. Microbial oil according to para 1 or 2, wherein a content of saturated fatty acids is 40 area% or less based on the total fatty acids.
4. Microbial oil according to any one of paras 1 to 3, wherein a content of arachidonic acid (ARA) is 10.0 area% or less based on the total fatty acids.
5. Microbial oil according to any one of paras 1 to 4, wherein the content of arachidonic acid (ARA) is 5.0 or less based on the content of EPA.
6. Microbial oil according to any one of paras 1 to 5, wherein a content of docosahexaenoic acid is lower than the content of eicosapentaenoic acid.
7. Microbial oil according to any one of paras 1 to 6, wherein the content of docosahexaenoic acid is 0.5 area% or more and 25 area% or less based on the total fatty acids.
8. Microbial oil according to any one of paras 1 to 7, wherein a content of n-6 docosapentaenoic acid (n-6DPA) is 2.0 area% or less based on a content of n-3 docosapentaenoic acid (n-3DPA).
9. Microbial oil according to any one of paras 1 to 8, wherein the content of n-3 docosapentaenoic acid (n-3DPA) is 0.3 area% or more based on the total fatty acids.
10. Microbial oil according to any one of paras 1 to 9, wherein a content of eicosatetraenoic acid (ETA) is 0.1 area% or more based on the total fatty acids.
11. Microbial oil according to any one of paras 1 to 10, wherein a total content of unsaturated fatty acids having 18 carbon atoms is 2.0 area% or less based on the total fatty acids.
12. Microbial oil according to any one of paras 1 to 11, which is a crude oil.
13. A method for producing a microbial oil comprising:
   preparing a microorganism having an ability to produce both eicosapentaenoic acid and docosahexaenoic acid;
   culturing the prepared microorganism in a medium containing glycerol under an environment aerated at 30 mL/min or more and 1500 mL/min or less; and
   obtaining a microbial oil as described in any one of paras 1 to 12 from biomass obtained after culturing.
14. Method for producing a microbial oil according to para 13, wherein the microorganism is at least one microorganism selected from the group consisting of microorganisms belonging to Stramenopiles.
15. Method for producing a microbial oil according to any one of para 13 or para 14, wherein the microorganism does not grow, or can produce more docosahexaenoic acid than eicosapentaenoic acid, in a medium containing glucose.
16. Method for producing a microbial oil according to any one of paras 13 to 15, wherein the culture environment is an environment equivalent to a water evaporation amount of 0.5 mL when the culture is left at 30°C for 10 days.
17. A microbial oil obtained by a production method described in any one of paras 13 to 16.
18. A method for controlling a fatty acid content ratio of a microbial oil, comprising:
   preparing a microorganism that does not grow, or can produce a microbial oil containing more docosahexaenoic acid than eicosapentaenoic acid, in a medium containing glucose;
   culturing the prepared microorganism in a culture medium containing glycerol under an environment aerated at 30 mL/min or more and 1500 mL/min or less; and
   obtaining a microbial oil containing more eicosapentaenoic acid than docosahexaenoic acid from biomass obtained after culturing.

## Claims

1. A microbial oil comprising eicosapentaenoic acid (EPA) and docosahexaenoic acid (DHA),
the content of eicosapentaenoic acid being 45 area% or greater based on the total fatty acids, and
the microbial oil having at least one fatty acid ratio selected from (a) to (c):
(a) a ratio of the content of EPA to the content of DHA of 1.7 or higher;
(b) a ratio of a content of fatty acids having 18 carbon atoms to the content of fatty acids having 20 carbon atoms of 0.3 or less;
(c) a ratio of a content of fatty acids having 18 carbon atoms to a content of fatty acids having 22 carbon atoms of 1.5 or less.

2. Microbial oil according to claim 1, wherein a content of triglyceride is 70% by weight or more based on a total weight of the microbial oil.

3. Microbial oil according to claim 1 or 2, wherein a content of saturated fatty acids is 40 area% or less based on the total fatty acids.

4. Microbial oil according to any one of claims 1 to 3, wherein a content of arachidonic acid (ARA) is 10.0 area% or less based on the total fatty acids.

5. Microbial oil according to any one of claims 1 to 4, wherein the content of arachidonic acid (ARA) is 5.0 or less based on the content of EPA.

6. Microbial oil according to any one of claims 1 to 5, wherein a content of docosahexaenoic acid is lower than the content of eicosapentaenoic acid.

7. Microbial oil according to any one of claims 1 to 6, wherein the content of docosahexaenoic acid is 0.5 area% or more and 25 area% or less based on the total fatty acids.

8. Microbial oil according to any one of claims 1 to 7, wherein a content of n-6 docosapentaenoic acid (n-6DPA) is 2.0 area% or less based on a content of n-3 docosapentaenoic acid (n-3DPA).

9. Microbial oil according to any one of claims 1 to 8, wherein the content of n-3 docosapentaenoic acid (n-3DPA) is 0.3 area% or more based on the total fatty acids.

10. Microbial oil according to any one of claims 1 to 9, wherein a content of eicosatetraenoic acid (ETA) is 0.1 area% or more based on the total fatty acids.

11. Microbial oil according to any one of claims 1 to 10, wherein a total content of unsaturated fatty acids having 18 carbon atoms is 2.0 area% or less based on the total fatty acids.

12. Microbial oil according to any one of claims 1 to 11, which is a crude oil.

13. A method for producing a microbial oil according to any one of claims 1 to 12, comprising:
preparing a microorganism having an ability to produce both eicosapentaenoic acid and docosahexaenoic acid;
culturing the prepared microorganism in a medium containing glycerol under an environment aerated at 30 mL/min or more and 1500 mL/min or less; and
obtaining a microbial oil as described in any one of claims 1 to 12 from biomass obtained after culturing;
wherein the microorganism is at least one microorganism selected from the group consisting of microorganisms belonging to Stramenopiles.

14. Method for producing a microbial oil according to claim 13, wherein the microorganism does not grow, or can produce more docosahexaenoic acid than eicosapentaenoic acid, in a medium containing glucose.

15. Method for producing a microbial oil according to any one of claims 13 to 14, wherein the culture environment is an environment equivalent to a water evaporation amount of 0.5 mL when the culture is left at 30°C for 10 days.
